# EUROPÄISCHE PATENTSCHRIFT

(11) **EP 3 463 212 B1**
(45) Veröffentlichungstag und Bekanntmachung des Hinweises auf die Patenterteilung: **18.12.2019**
(21) Anmeldenummer: 17726318.3
(22) Anmeldetag: 30.05.2017
(51) Int. Cl.: A61F 5/01

(54) **FUSSHEBERORTHESE**
FOOT LIFT ORTHOSIS
ORTHÈSE DE RELEVEUR DE PIED

(30) Priorität: 31.05.2016 DE 102016109963
(43) Veröffentlichungstag der Anmeldung: 10.04.2019
(73) Patentinhaber: Ottobock SE & Co. KGaA, 37115 Duderstadt (DE)
(72) Erfinder: HARDT, Alexander, 37115 Duderstadt (DE)
(74) Vertreter: Gramm, Lins & Partner Patent- und Rechtsanwälte PartGmbB
(86) Internationale Anmeldenummer: PCT/EP2017/062976
(87) Internationale Veröffentlichungsnummer: WO 2017/207532

(56) Entgegenhaltungen:
- WO-A1-2011/029837
- CN-Y- 201 414 857
- US-A1- 2008 077 066
- US-A1- 2015 190 263
- US-A1- 2016 067 075

## Beschreibung

Die Erfindung betrifft eine Fußheberorthese mit wenigstens einem ersten Zugelement, das eingerichtet ist, sich im angelegten Zustand der Fußheberorthese von einem Vorfußbereich eines Fußes entlang eines Fußrückens des Fußes zu erstrecken und eine erste Zugskraft auf den Vorfußbereich in Richtung auf eine erste Lagerposition oberhalb eines oberen Knöchelgelenks auszuüben.

Eine derartige Fußheberorthese ist beispielsweise aus der WO 2011/029837 A1 bekannt. Die US 2015/190263 A1 beschreibt eine Fußheberorthese, bei der sich das erste Zugelement von der Zehenspitze des Fußes direkt zu der ersten Lagerposition erstreckt. Dabei verläuft es auf direktem Weg und erstreckt sich nicht entlang des Fußrückens. Aus der CB201 414 857 Y ist eine Fußheberorthese bekannt, die aus einem elastischen Band besteht, das in Form einer Acht angeordnet ist.

Beispielsweise nach einem Schlaganfall aber auch aufgrund anderer Ursachen leiden Menschen oftmals unter einer Fußheberschwäche und der Fuß befindet sich in einer sogenannten Supinationsstellung. In diesem Fall kann die Fußspitze nicht mehr in ausreichendem Maße angehoben werden, so dass die Stolpergefahr deutlich erhöht ist.

Um dieser Schwäche entgegenzuwirken sind aus dem Stand der Technik eine Reihe unterschiedlicher Fußheberorthesen bekannt. Eine der konstruktiv einfachsten Fußheberorthesen besteht aus einer Manschette, die oberhalb des Knöchels um das Bein des Trägers gelegt wird und die über Gurte oder Zugelemente verfügt, die mit dem Schuh, insbesondere einer Schnürung eines Schuhs, verbunden wird. Dadurch wird auf den Schuh eine nach oben gerichtete Kraft ausgeübt, die naturgemäß auch den Fuß anhebt. Nachteilig ist jedoch, dass eine derartige Orthese nicht barfuß und auch nicht mit jedem Schuh getragen werden kann und zudem von außen durch Unbeteiligte leicht erkennbar ist.

Alternativ dazu kann das Zugelement, das von der oberhalb des Knöchels angeordneten Manschette ausgeht, auch an einer zweiten Manschette angeordnet sein, die im Vorfußbereich den Fuß umgibt. Durch das Zugelement wird folglich eine nahezu diagonal verlaufende Zugverbindung zwischen dem Fuß und dem Bein des Trägers gespannt, so dass eine derartige Fußheberorthese nicht mit einem Schuh tragbar ist. Hinzukommt, dass der Vorfuß durch die umlaufende Manschette, über die die Kraft auf den Fuß übertragen wird, in Umfangsrichtung komprimiert wird, was aus therapeutischer Sicht oftmals zu vermeiden ist. Eine nur auf den Vorfuß nach oben gerichtet ausgeübte Kraft führt zudem dazu, dass der Fuß nicht als Ganzes angehoben wird, sondern dass es zu einer "Verbiegung" des Fußes kommen kann, wodurch die Fußsohle gestreckt oder gar überstreckt wird. Auch dies ist nicht von Vorteil.

Eine andere Art von Fußheberorthesen verfügt über ein stabil ausgebildetes Sohlenelement, das sich über einen mehr oder weniger großen Anteil der Fußsohle erstreckt. Es besteht beispielsweise aus Kohlefaserverbundwerkstoff und ist mit einer Stützeinrichtung verbunden, die in der Regel am Unterschenkel des Patienten angeordnet wird. Durch die stabile Sohlenplatte, die unter dem Fuß entlang läuft, wird der Fuß und damit die Fußspitze angehoben. Zur Abstützung benötigen die meisten dieser Fußheberorthesen jedoch einen umgebenen Schuh und sind insbesondere nicht barfuß zu tragen, da sie einerseits von außen leicht erkennbar sind und andererseits ein barfuß-Tragegefühl nicht erreicht werden kann, da die Fußsohle zu einem großen Anteil durch das Sohlenelement abgedeckt ist.

Der Erfindung liegt daher die Aufgabe zugrunde, eine Fußheberorthese vorzuschlagen, die auch barfuß zu tragen ist, die leicht zu reinigen ist und dennoch kostengünstig und einfach herstellbar ist.

Die Erfindung löst die gestellte Aufgabe durch eine Fußheberorthese gemäß dem Oberbegriff des Anspruchs 1, die sich dadurch auszeichnet, dass sie wenigstens ein zweites Zugelement aufweist, das eingerichtet ist, im angelegten Zustand der Fußheberorthese eine zweite Zugkraft auf den Vorfußbereich in Richtung auf eine zweite Lagerposition in einem Fersenbereich des Fußes auszuüben.

Vorteilhafterweise ist das zweite Zugelement eingerichtet, sich im angelegten Zustand der Fußheberorthese von dem Vorfußbereich entlang einer Fußsohle des Fußes zu erstrecken.

Die erfindungsgemäße Fußheberorthese verfügt folglich über zwei Zugelemente, von denen das erste Zugelement über den Fußrücken und das zweite Zugelement vorzugsweise entlang der Fußsohle verläuft. Beide greifen mit jeweils einem Ende am Vorfußbereich, beispielsweise im Bereich des Ballens des Fußes an und üben auf diesen Vorfußbereich eine Zugkraft aus. Das erste Zugelement übt eine Zugkraft aus, die in Richtung auf die erste Lagerposition gerichtet ist, die sich oberhalb des oberen Knöchelgelenkes befindet. Dadurch wird der Fußbereich angehoben und so die Funktion der Fußheberorthese gewährleistet. Gleichzeitig übt das zweite Zugelement eine zweite Zugkraft aus, die nahezu oder vollständig parallel zur Fußsohle verläuft und so eine Biegung des Fußes innerhalb des Fußes verhindert. Da beide aufgebrachten Zugkräfte im Vorfußbereich des Fußes angreifen, erfolgt durch diese Kräfte ein "Kompaktierung" des Fußes. Beide Kräfte gemeinsam sorgen folglich dafür, dass der Fuß unter einer in Richtung auf die Ferse gerichteten Kraft gehalten wird, so dass es insbesondere zu einer Spannung der Plantaraponeurose, also der Sehnenplatte des Fußes, kommt. Eine gegebenenfalls negative Verbiegung des Fußes durch auf den Vorfuß wirkende nach oben gerichtete Kräfte, wie dies aus dem Stand der Technik gegebenenfalls bekannt ist, wird auf diese Weise ausgeschlossen.

Da das erste Zugelement sich entlang des Fußrückens und das zweite Zugelement sich vorzugsweise entlang der Fußsohle des Fußes des Trägers erstreckt, trägt die Orthese kaum auf und kann sowohl mit als auch ohne Schuh, insbesondere auch barfuß, verwendet werden. Da die Fußheberorthese nahezu vollflächig am Fuß anliegt, ist sie, sofern sie aus einem transparenten oder hautfarbenen Material hergestellt wird, nicht auf den ersten Blick als Orthese erkennbar, so dass sie auch barfuß unauffällig getragen werden kann.

Vorteilhafterweise sind das erste Zugelement und das zweite Zugelement im Vorfußbereich miteinander verbunden und vorzugsweise einstückig miteinander ausgebildet. Diese einstückige Ausgestaltung kann beispielsweise aus Silikon bestehen. Dabei ist es jedoch möglich, die beiden Zugelemente unterschiedlich dick oder unterschiedlich breit auszubilden, und so selbst bei der Verwendung des identischen Materials und der einstückigen Ausbildung der beiden Zugelemente unterschiedlich starke Zugkräfte zu erzeugen. Selbstverständlich ist es auch möglich eines oder beide der Zugelemente aus Silikon zu fertigen und diese auf eine andere Art im Vorfußbereich miteinander zu verbinden.

Vorzugsweise verfügt das zweite Zugelement über eine Öffnung, so dass es um die Ferse des Fußes herumlegbar ist. In diesem Fall ist das Anlegen der Fußheberorthese im Bereich des zweiten Zugelementes besonders einfach, da das zweite Zugelement lediglich im Vorfußbereich angreifen muss und dann die Ferse des Trägers durch die Öffnung im zweiten Zugelement hindurchgeführt werden kann. Auf diese Weise dient die Ferse als Gegenlager für die zweite Zugkraft, die durch das zweite Zugelement aufgebracht wird. Alternativ oder zusätzlich dazu ist es möglich, eine Umschließung des Fußes vorzusehen, die so als Widerlager für die zweite Zugkraft wirkt. Es kann eine Lasche oder ein anderes Griffelement vorhanden sein, durch das das Anlegen der Orthese weiter vereinfacht wird.

Vorteilhafterweise verfügt das erste Zugelement über wenigstens eine Öffnung, so dass um einen Knöchelbereich herumlegbar ist. Das erste Zugelement verläuft dann oberhalb des Knöchels einmal um das Bein des Trägers herum, so dass dieses als Widerlager für die erste Zugkraft dient. Zum Anlegen der Fußheberorthese wird einfach der Fuß durch diese Öffnung im ersten Zugelement hindurchgeführt, ein Verbindungsbereich zwischen dem ersten Zugelement und dem zweiten Zugelement im Vorfußbereich des Fußes angeordnet und anschließend vorteilhafterweise die Ferse durch die Öffnung im zweiten Zugelement hindurchgeführt. Es müssen keine Riemen oder Gurte geschlossen oder Schnürsenkel oder Seile geschnürt werden, so dass das Anlegen der Fußheberorthese einfach und reproduzierbar möglich ist.

Vorzugsweise folgt das erste Zugelement dem Verlauf der Plantaraponeurose. Besonders vorteilhaft wird dabei nur ein kleiner Teil der Fußsohle, insbesondere des Ballenbereiches abgedeckt, um dem Träger möglichst das Gefühl zu geben, tatsächlich barfuß zu laufen, wenn die Fußheberorthese direkt am Fuß, also ohne Socken, Strumpf und Schuh getragen wird.

In einer besonders bevorzugten Ausführungsform verfügt die Fußheberorthese über wenigstens einen Haltesteg, der sich von der ersten Lagerposition aus nach oben erstreckt und um den Unterschenkel des Trägers herumführbar ist. Dies geschieht insbesondere im posterioren Bereich des Beines des Trägers. Dadurch wird die durch das erste Zugelement aufgebrachte Zugkraft nach posterior umgelenkt. Der Haltesteg verläuft im angelegten Zustand der Fußheberorthese am Unterschenkel des Trägers nach oben und wird dort um den Unterschenkel herumgeführt. Auch dadurch kann die durch das erste Zugelement aufgebrachte Zugkraft verändert, und insbesondere verstärkt werden. Vorteilhafterweise ist die Umschließung um den Unterschenkel, die Teil des Haltesteges ist, so ausgebildet, das sie reversibel geöffnet und geschlossen werden kann. Dies kann beispielsweise über einen Druckknopf oder ein Formschlusselement, beispielsweise über Klettverschlüsse, erfolgen. Dadurch wird das Anlegen und Ablegen der Fußheberorthese deutlich vereinfacht.

Vorteilhafterweise befindet sich zwischen dem ersten Zugelement und dem zweiten Zugelement wenigstens eine Öffnung, durch die sich im angelegten Zustand wenigstens ein Zeh des Fußes erstreckt. Natürlich kann auch eine Öffnung vorhanden sein, durch die sich mehr als ein Zeh erstreckt und/oder es sind wenigstens eine Öffnung, vorteilhafterweise mehrere Öffnungen vorhanden, durch die sich jeweils ein Zeh erstreckt. Dies geschieht im Verbindungsbereich zwischen dem ersten Zugelement und dem zweiten Zugelement, der als separates Bauteil, das mit dem ersten Zugelement und dem zweiten Zugelement verbunden ist, oder als Übergangsbereich zwischen den beiden Zugelementen ausgebildet sein kann. Selbstverständlich ist auch eine einstückige Ausgestaltung mit dem ersten Zugelement und dem zweiten Zugelement denkbar, so dass beispielsweise die Öffnung in einem Silikonelement vorhanden ist. Auf diesen Übergangsbereich zwischen dem ersten Zugelement und dem zweiten Zugelement wird durch die beiden Zugelemente die jeweils aufzubringende Zugkraft aufgebracht, so dass es durch die Öffnung, durch die sich wenigstens ein Zeh des Fußes erstreckt, auch zu einer Zehenfixierung kommt. Selbstverständlich ist es auch möglich, dass sich in diesem Bereich eine Öffnung befindet, durch die sämtliche Zehen hindurch geführt werden, so dass sich die Fußheberorthese im Vorfußbereich ausschließlich am Ballen des Fußes abstützt.

Vorzugsweise verfügt die Fußheberorthese über wenigstens ein Verstärkungselement, das an dem Unterschenkel befestigbar ist und sich entlang des Fußrückens erstreckt, so dass die von dem ersten Zugelement aufbringbare erste Zugkraft verstärkbar ist. Das wenigstens eine Verstärkungselement ist insbesondere ein separates Bauteil, so dass es leicht gegen ein anderes Verstärkungselement austauschbar ist. Dadurch kann die insgesamt durch die Fußheberorthese aufbringbare Kraft besonders einfach individuell auf die Bedürfnisse des Trägers eingestellt und gegebenenfalls im Verlauf einer Therapie an geänderte Situationen, beispielsweise eine fortschreitende Heilung, angepasst werden. Das wenigstens eine Verstärkungselement kann an seinem einen Ende über eine Befestigungseinrichtung verfügen, über die es am Unterschenkel des Trägers befestigt werden kann. Auch dies erfolgt durch eine Umschließung, die durch ein Verschlusselement lösbar geschlossen werden kann. Am entgegengesetzten Ende des Verstärkungselementes kann dieses über Formschlusselemente oder auf andere Weise am ersten Zugelement angeordnet sein. Dies geschieht vorteilhafterweise möglichst nah am Vorfußbereich des Fußes, so dass auch durch das Verstärkungselement eine Zugkraft auf diesen Vorfußbereich ausgeübt werden kann. Es hat sich als vorteilhaft herausgestellt, wenn das Verstärkungselement so am Unterschenkel des Trägers angeordnet wird, das es vom Haltesteg und der zu ihm gehörenden Umschließung des Unterschenkels mit umschlossen wird, um zu gewährleisten, dass die Fußheberorthese insgesamt möglichst wenig aufträgt.

Bevorzugt verfügt die Fußheberorthese über wenigstens ein Stabilisierungselement, das sich im angelegten Zustand um den Vorfußbereich herumerstreckt.

In einer besonders einfach herzustellenden und besonders leicht zu reinigenden Ausgestaltung besteht die Fußheberorthese aus Silikon und ist vorteilhafterweise einstückig ausgebildet. Die gesamte Fußheberorthese besteht dann nur aus einem einzigen Silikonelement, in dem sich vorteilhafterweise die benötigten Öffnungen befinden. Dies ist im nicht angelegten Zustand besonders klein zusammenlegbar, so dass es einfach zu lagern und zu transportieren ist und ist zudem leicht anzulegen und insbesondere gegen Salzwasser unempfindlich und leicht zu reinigen. Dabei kann ein Verstärkungselement weiterhin als separates Bauteil ausgebildet sein. Es ist vorzugsweise aus dem gleichen Material wie der Rest der Fußheberorthese hergestellt.

Wenn die Fußheberorthese ein separates Element ist, das insbesondere aus Silikon besteht und aus einem einzelnen Werkstück hergestellt ist, ist es von Vorteil, wenn die Fußheberorthese individuell anpassbar ist. Selbstverständlich ist dies auch bei mehrteilig ausgebildeten Fußheberorthesen oder anders ausgebildeten Fußheberorthesen von Vorteil.

Diese individuelle Anpassbarkeit kann beispielsweise erreicht werden, indem beispielsweise Öffnungen oder Löcher, die in der Fußheberorthese vorzusehen sind, individuell hergestellt und in die Fußheberorthese eingeschnitten werden können. Dies kann beispielsweise eine Ausnehmung oder Öffnung sein, durch die im angelegten Zustand der Fußheberorthese die Ferse hindurchragt. Eine weitere Öffnung, die vorzugsweise individuell ausgestaltbar ist, ist im angelegten Zustand für die Zehen des Fußes gedacht. Auch die Einstiegsöffnung, durch die der Fuß hindurchgeführt wird, wenn die Fußheberorthese angelegt wird, ist vorzugsweise auf diese Weise individualisierbar. Vorzugsweise kann die Position und/oder die Größe wenigstens einer dieser Öffnungen, bevorzugt jedoch mehrerer oder aller dieser Öffnungen angepasst werden. Auf diese Weise kann beispielsweise auf unterschiedlich große Füße reagiert werden, indem beispielsweise der Abstand zwischen der Öffnung für die Ferse und der Öffnung für die Zehen vergrößert oder verkleinert werden kann. In einer bevorzugten Ausgestaltung werden die benötigten Öffnungen beispielsweise mittels einer Schere oder einem anderen Schneidwerkzeug in das Material der Fußheberorthese eingeschnitten. Dazu können Markierungslinien auf der Fußheberorthese vorhanden sein, die beispielsweise bestimmte Schuhgrößen markieren, für die die Orthese passend eingerichtet werden soll. Ein Orthopädietechniker oder der Träger der Orthese muss dann nur entlang der für ihn passenden Linien die gewünschten Öffnungen in das Material der Fußheberorthese einschneiden und kann auf diese Weise eine individuell für ihn hergestellte Fußheberorthese generieren. Insbesondere lässt sich auch auf körperliche Besonderheiten und individuelle Gegebenheiten am jeweiligen Fuß eingehen.

Neben der Größe und besonderen körperlichen Eigenschaften des jeweiligen Fußes ist es auch möglich, die Fußheberorthese auf diese Weise so bequem wie möglich auszugestalten, indem beispielsweise das Loch für die Zehen in der Größe variiert werden kann. So ist es möglich, mehrere Löcher für jeweils einen Zeh oder ein Loch für mehrere Zehen in das Material der Fußheberorthese einzuschneiden. Selbstverständlich können auch Kombinationen dieser Lochkonfigurationen verwendet werden.

Alternativ dazu ist es auch möglich, dass die Fußheberorthese einen Strumpf aus einem textilem Material aufweist, an dem das erste Zugelement und das zweite Zugelement angeordnet, bevorzugt angeklebt oder angespritzt sind. Dieser Strumpf ist vorzugsweise auf einer Innenseite, die dem Fuß zugewandt ist, mit einer rutschhemmenden Beschichtung versehen. Die Beschichtung kann vollflächig oder bereichsweise, vorzugsweise im Fersenbereich, im Knöchelbereich und/oder im Vorfußbereich, aufgebracht sein. Bevorzugt wird eine Beschichtung mit Silikon.

Mit Hilfe der beiliegenden Figuren wird nachfolgend ein Ausführungsbeispiel der vorliegenden Erfindung näher erläutert. Es zeigt:
- Figur 1: - die schematische Ansicht einer Fußheberorthese gemäß einem ersten Ausführungsbeispiel der vorliegenden Erfindung in einer Seitenansicht,
- Figur 2: - die schematische Darstellung einer Fußheberorthese in einer 3D-Ansicht,
- Figur 2a: - eine leichte Abwandlung der in Figur 2 gezeigten Ausführungs-form,
- Figur 3: - die schematische Darstellung der Fußheberorthese aus Figur 2 in einer Draufsicht,
- Figur 4: - die Fußheberorthese in einer Ansicht von unten,
- Figur 5: - die schematische Darstellung der Fußheberorthese in einer Rückansicht,
- Figur 6: - die schematische Darstellung der Fußheberorthese im nicht angelegten Zustand,
- Figur 7: - die schematische Darstellung einer Fußheberorthese gemäß einem weiteren Ausführungsbeispiel der vorliegenden Erfindung,
- Figur 8: - die schematische Darstellung einer Fußheberorthese gemäß einem weiteren Ausführungsbeispiel der vorliegenden Erfindung,
- Figuren 9-11: - schematische Ansichten einer Fußheberorthese gemäß einem weiteren Ausführungsbeispiel der vorliegenden Erfindung,
- Figuren 12 - 14: - ein weiteres Ausführungsbeispiel und
- Figur 15: - die schematische Darstellung eines weiteres Ausführungsbeispiels.

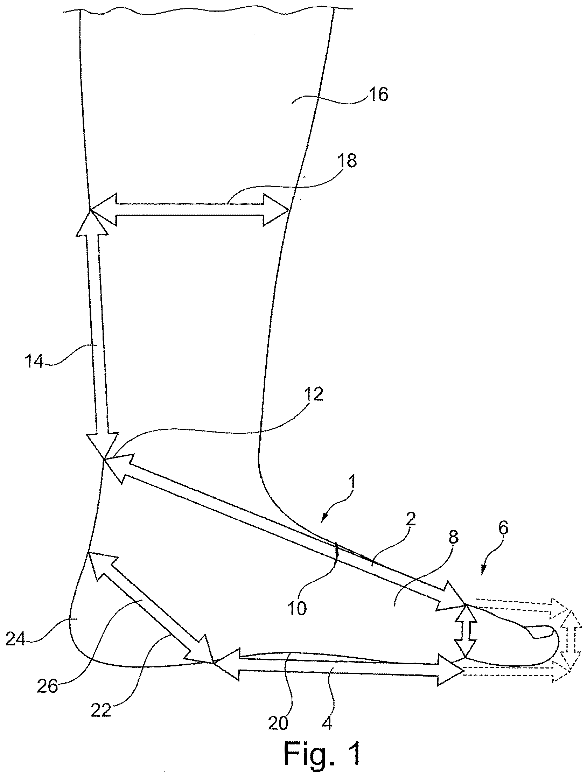

Figur 1 zeigt die schematische Darstellung einer Fußheberorthese 1. Sie verfügt über ein erstes Zugelement 2 und ein zweites Zugelement 4, die jeweils mit einem Ende an einem Vorfußbereich 6 eines Fußes 8 angreifen. Das erste Zugelement 2 erstreckt sich vom Vorfußbereich 6 entlang eines Fußrückens 10 zu einer ersten Lagerposition 12, die sich oberhalb des oberen Knöchelgelenkes befindet. Auf diese Weise kann eine erste Zugkraft auf den Vorfußbereich 6 ausgeübt werden, die sich entlang des ersten Zugelementes 2 erstreckt. Am posterioren Ende des ersten Zugelementes 2 schließt sich ein Haltesteg 14 an, der entlang des Unterschenkels 16 nach oben verläuft und eine Umfassung 18 aufweist, die um den Unterschenkel 16 herumlegbar ist. Dabei verfügt die Umfassung 18 vorzugsweise über ein in Figur 1 nicht dargestelltes Verschlusselement, so dass die Umfassung 18 zum Anlegen und Ablegen der Fußheberorthese geöffnet werden kann.

Das zweite Zugelement 4, das ebenfalls im Vorfußbereich 6 des Fußes 8 angreift, erstreckt sich entlang einer Fußsohle 20 bis zu einer zweiten Lagerposition 22 im Bereich der Ferse 24 des Fußes 8. Das zweite Zugelement 4 verfügt über eine Öffnung 26, durch die sich die Ferse 24 erstreckt, so wie auch das erste Zugelement 2 im in Figur 1 gezeigten Ausführungsbeispiel eine in der Figur nicht gezeigte Öffnung aufweist, durch die sich der Unterschenkel 16 erstreckt.

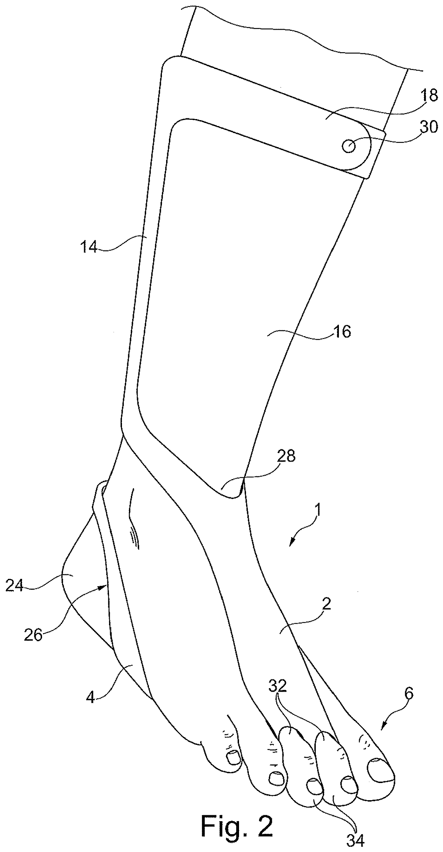

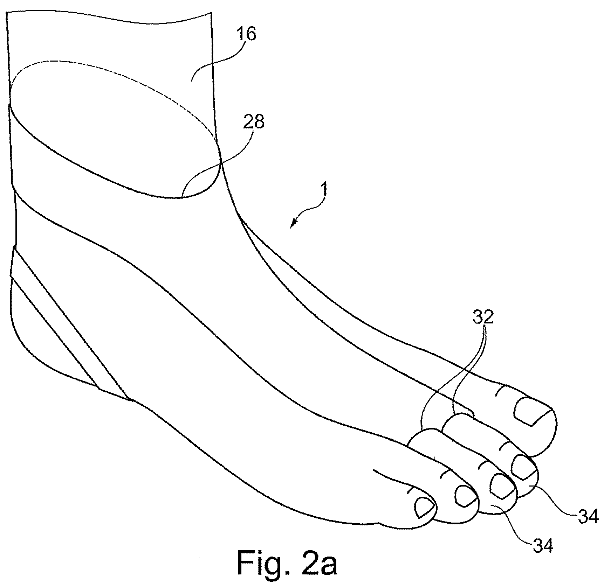

Figur 2 zeigt die Fußheberorthese 1 in einer schematischen dreidimensionalen Ansicht. Man erkennt das erste Zugelement 2 sowie einen Teil des zweiten Zugelementes 4, durch dessen Öffnung 26 sich die Ferse 24 erstreckt. Die Öffnung 28 im ersten Zugelement 2 ist nun zu erkennen, durch die sich der Unterschenkel 16 erstreckt. Im Posteriorbereich des Unterschenkels 16 ist der Haltesteg 14 mit der sich daran anschließenden Umfassung 18 zu erkennen, die nun über ein Verschlusselement 30 verfügt, das im vorliegenden Beispiel als Dorn ausgebildet ist, der durch ein Loch geführt wird.

Im Vorfußbereich 6 sind zwei Öffnungen 32 dargestellt, durch die sich jeweils ein Zeh 34 erstreckt.

In Figur 2a ist eine Ausgestaltung der Fußheberorthese 1 gezeigt, die im Wesentlichen der in Figur 2 gezeigten Ausführungsform entspricht. Durch die Öffnung 28 erstreckt sich der Unterschenkel 16, während durch die Öffnungen 32 die Zehen 34 hindurchragen. Anders als die in Figur 2 gezeigte Ausführungsform verfügt die in Figur 2a gezeigte Fußheberorthese 1 jedoch nicht über einen Haltesteg 14 und eine Umfassung 18, durch die eine weitere Halteposition am Unterschenkel 16 erreicht wird.

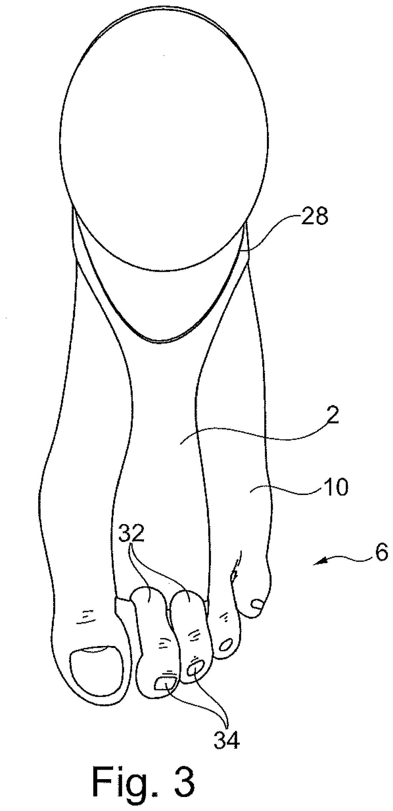

Figur 3 zeigt die in Figur 2 gezeigte Orthese in einer Draufsicht. Das erste Zugelement 2 erstreckt sich über den Fußrücken 10 von einem Vorfußbereich 6 in Richtung auf den Knöchelbereich und verfügt über die Öffnung 28, durch die das Bein geführt ist. Im Vorfußbereich 6 sind die beiden Öffnungen 32 zu erkennen, durch die sich die Zehen 34 erstrecken.

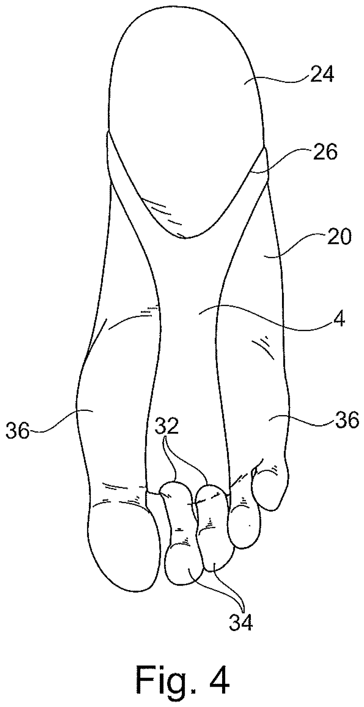

Figur 4 zeigt die Fußheberorthese 1 in einer Ansicht von unten. Man erkennt die Fußsohle 20 und das zweite Zugelement 4, wobei sich durch dessen Öffnung 26 die Ferse 24 erstreckt. Ein Großteil eines Ballens 36 wird vom zweiten Zugelement 4 nicht abgedeckt, so dass der Träger einer Orthese ein barfuß-Gefühl erleben kann, wenn er die Orthese 1 in der gezeigten Ausführungsform ohne Socken und Schuhe trägt. Durch die Öffnungen 32 erstrecken sich wie in Figur 3 zwei Zehen 34.

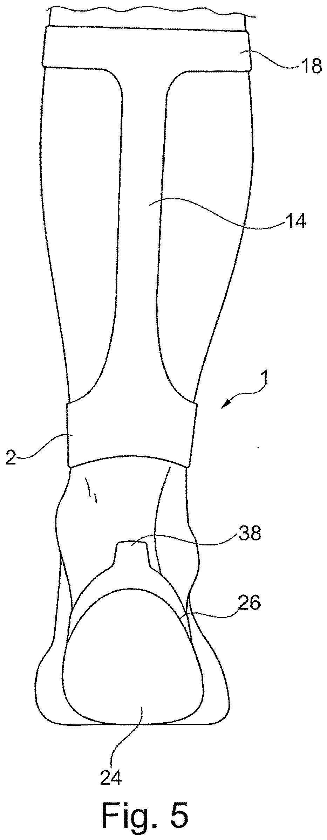

Figur 5 zeigt die Fußheberorthese 1 in einer Rückansicht. Die Ferse 24 erstreckt sich durch die Öffnung 26 im zweiten Zugelement 4. An ihm befindet sich eine Lasche 38, durch die das Anlegen erleichtert wird, da die Lasche 38 gegriffen und so das zweite Zugelement 4 gespannt und die Ferse 24 durch die Öffnung 26 hindurchgeführt werden kann. Wie bereits dargelegt schließt sich am ersten Zugelement 2 der Haltesteg 14 mit der Umfassung 18 an.

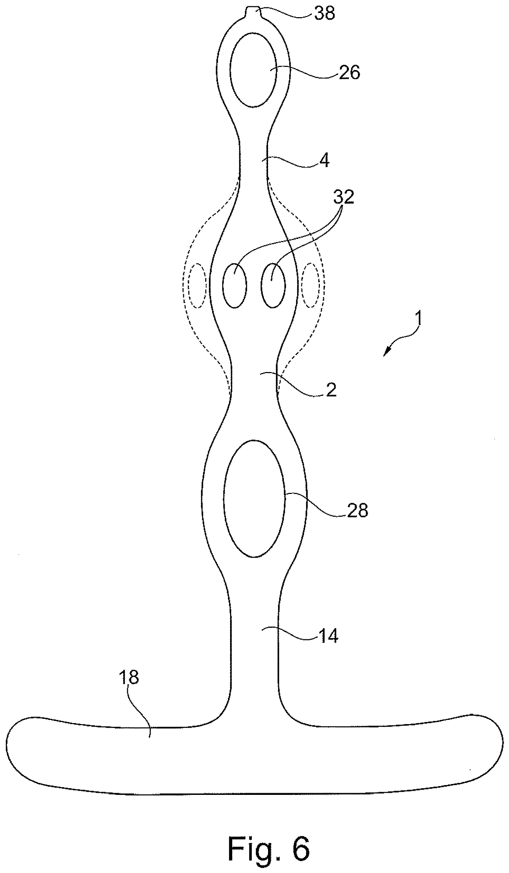

Figur 6 zeigt die Fußheberorthese 1 im nicht angelegten Zustand. Die Fußheberorthese 1 wie in Figur 6 dargestellt ist einstückig ausgebildet und besteht vorteilhafterweise aus Silikon. Diese Ausführungsform ist besonders leicht zu reinigen, einfach herzustellen und insbesondere wasserfest, was insbesondere auch für Seewasser, also Salzwasser gilt. Man erkennt deutlich, dass sich zwischen dem ersten Zugelement 2 und dem zweiten Zugelement 4 die Öffnungen 32 für die Zehen befinden. In einer alternativen Ausführungsform der Fußheberorthese 1, die in Figur 6 mit gestrichelten Linien dargestellt ist, ist die Anzahl der Öffnungen 32 auf 4 erhöht worden, so dass vier Zehen durch jeweils eine diese Öffnungen 32 geführt werden können. Natürlich ist es auch möglich, die Öffnungen 32 so auszubilden, dass mehr als jeweils ein Zeh hindurchgeführt werden kann.

An dem den Öffnungen 32 gegenüberliegenden Ende des zweiten Zugelementes 4 befindet sich dessen Öffnung 26, durch die die Ferse 24 geführt werden kann. An den Öffnungen 32 gegenüberliegenden Ende des ersten Zugelementes 2 befindet sich die Öffnung 28. Zum Anlegen der Orthese wird nun der Fuß durch die Öffnung 28 hindurchgeführt und anschließend der Bereich zwischen den beiden Zugelementen 2, 4, in denen sich die Öffnungen 32 befinden, an den Zehen und im Vorfußbereich 6 des Fußes angeordnet. Dadurch wird das erste Zugelement 2 gespannt und bereits eine Zugkraft auf den Vorfuß 6 ausgeübt. Anschließend kann das zweite Zugelement 4 entlang der Fußsohle gespannt und die Ferse 24 durch die Öffnung 26 hindurchgeführt werden. Dazu kann die Lasche 38 gegriffen werden, wodurch das Anlegen der Fußheberorthese 1 deutlich vereinfacht wird. Zum Anlegen wird zudem der Haltesteg 14 entlang des Unterschenkels angeordnet und die Umschließung 18 geschlossen.

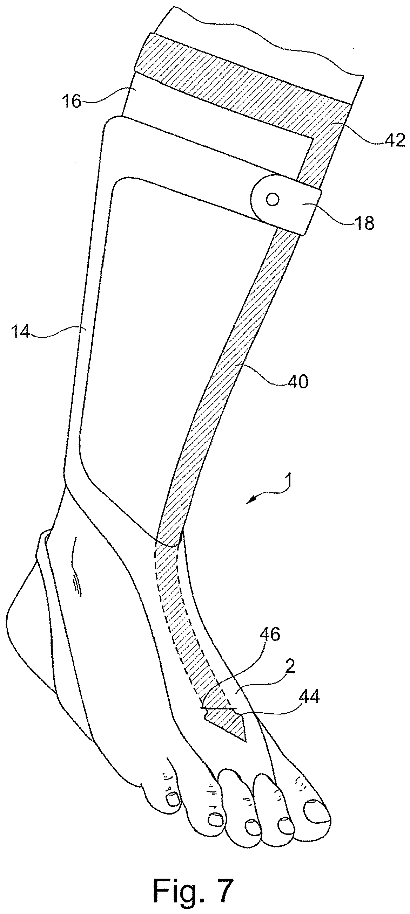

Figur 7 zeigt die Fußheberorthese 1 gemäß einem weiteren Ausführungsbeispiel der vorliegenden Erfindung. Neben dem ersten Zugelement 2 und dem sich daran anschließenden Haltesteg 14 mit der Umschließung 18 verfügt die in Figur 7 gezeigte Fußheberorthese über ein Verstärkungselement 40, das im oberen Bereich durch eine weitere Umschließung 42 am Unterschenkel 16 des Trägers der Fußheberorthese befestigt werden kann. Im unteren Bereich des Verstärkungselementes 40 ist es durch ein im gezeigten Ausführungsbeispiel pfeilspitzenförmiges Formschlusselement 44 am ersten Zugelement 2 befestigt. Dazu wird das Formschlusselement 44 durch einen im ersten Zugelement 2 vorhandenen Schlitz 46 hindurchgeführt. Auch das Verstärkungselement ist vorteilhafterweise elastisch ausgebildet. Doch auch durch ein nicht elastisch ausgebildetes Verstärkungselement 40 lässt sich eine zusätzliche Zugkraft auf den Vorfußbereich aufbringen, die die Zugkraft des ersten Zugelementes 2 verstärkt.

Die insbesondere in Figur 6 gezeigte besondere Schnittform der vorzugsweise einstückig ausgebildeten Fußheberorthese ermöglicht ein leichtes Anziehen der Orthese. Durch das zweite Zugelement 4, das der Plantaraponeurose folgt und der Öffnung 26, die einen ringförmigen Halt um die Ferse 24 ermöglicht, wird eine Gegenspannung zum dorsalen Zug, also der ersten Zugkraft, die durch das erste Zugelement 2 aufgebracht wird, erreicht. Dieser dorsale Zug, der im gezeigten Ausführungsbeispiel durch die Zehen 34 und anschließend ringförmig über das obere Sprunggelenk, also die erste Lagerposition 12 verläuft, ermöglicht die Fußhebung. Durch unterschiedliche Festigkeiten, Materialstärken, Breiten oder sonstige Änderungen in den Parametern können durch das erste Zugelement 2 und das zweite Zugelement 4 unterschiedlich starke Zugkräfte aufgebracht werden. Um ein Verrutschen der einzelnen Elemente am Fuß zu verhindern und gegebenenfalls auch die Zugrichtung noch zu verstärken führt der Haltesteg 14 posterior an der Achillessehne nach proximal und wird beispielsweise in einem Abstand von circa 15 bis 20 cm mit der Umfassung 18 unterhalb der Wade fixiert. Auch dies geschieht vorteilhafterweise vollständig mit wasserfesten und insbesondere auch durch Salzwasser nicht angreifbaren Materialien.

Durch den Haltesteg bleibt der Verschluss der Umfassung 18 an seinem Platz und kann nicht in Richtung des Tal abrutschen.

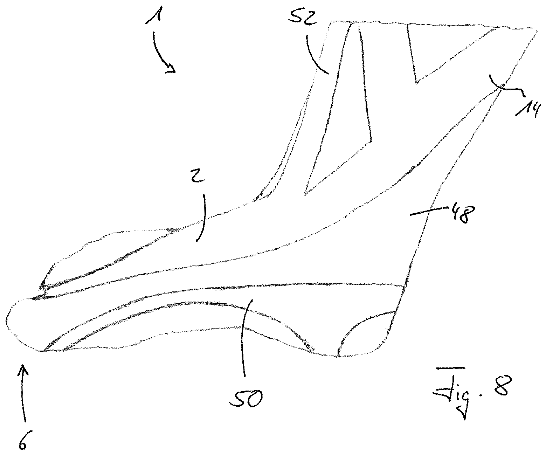

Figur 8 zeigt eine weitere Ausgestaltung der Fußheberorthese 1 gemäß einem weiteren Ausführungsbeispiel der vorliegenden Erfindung. Verschiedene Bahnen 50 aus dem Material der Fußheberorthese 1 sind auf einen Strumpf 48 aufgebracht, beispielsweise angeklebt oder angespritzt worden. Man erkennt das erste Zugelement 2, das im Vorfußbereich 6 um die Zehen herum geführt wird. Der Haltesteg 14 ist im gezeigten Beispiel kreuzförmig ausgebildet. Zudem sind weitere Stabilisierungselemente 52 vorhanden. Durch die Ausgestaltung als Strumpf 48 wird ein Verrutschen der Fußheberorthese 1 am Fuß 8 weitgehend verhindert, so dass die zur Stabilisierung verwendeten Elemente 14, 52 und andere nicht gezeigte Bauteile anders ausgebildet sein können.

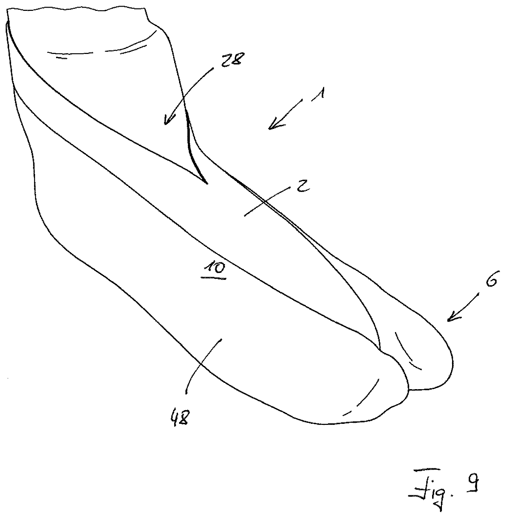

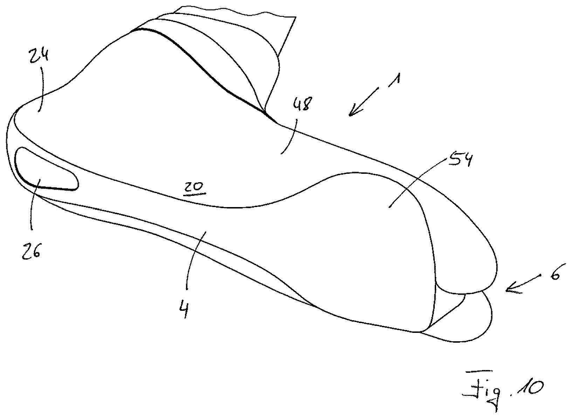

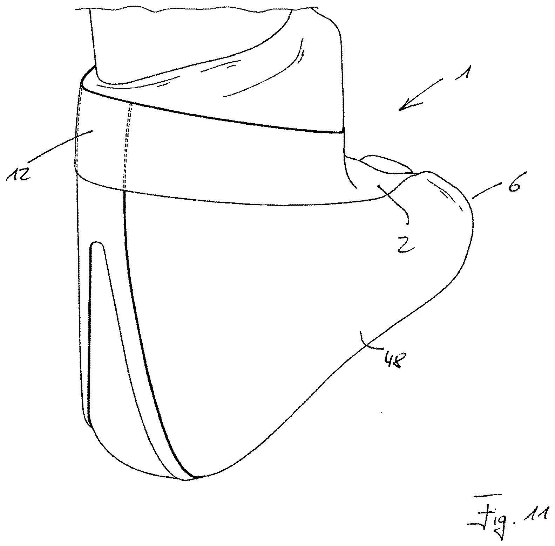

In den Figuren 9 bis 11 ist eine besonders einfache Ausgestaltung der Fußheberorthese 1 dargestellt. Man erkennt den Strumpf 48 auf den das Material der Fußheberorthese 1 aufgebracht ist. Das erste Zugelement 2 erstreckt sich über den Fußrücken 10 bis in den Vorfußbereich 6. Durch die Öffnung 28 wird der Fuß hindurchgeführt und die erste Lagerposition 12 im hinteren Bereich des Fußes, im gezeigten Ausführungsbeispiel im Bereich der Achillessehne, gebildet. Im Bereich der Fußsohle 20 erstreckt sich das zweite Zugelement 4 aus dem Vorfußbereich 6 bis zur Ferse 24, wo sich die Öffnung 26 befindet. Im Bereich des Ballens des Fußes befindet sich eine Verbreiterung 54 aus dem Material der Fußheberorthese 1, das beispielsweise Silikon sein kann. Dadurch wird insbesondere in diesem Bereich, in dem eine hohe Belastung auf den Fuß wirkt, wenn dieser aufgesetzt wird, eine möglichst gleichmäßige Verteilung des Orthesenmaterials gewährleistet, wodurch unbequeme oder schmerzhafte Druckstellen vermieden werden können.

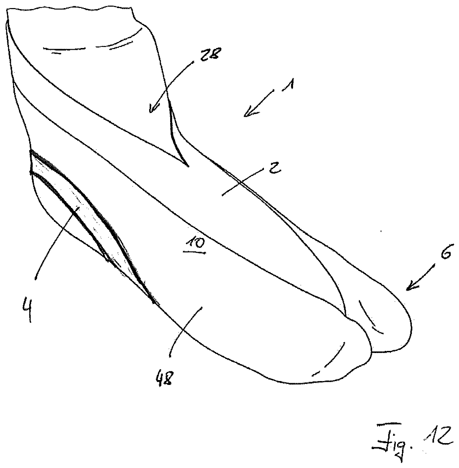

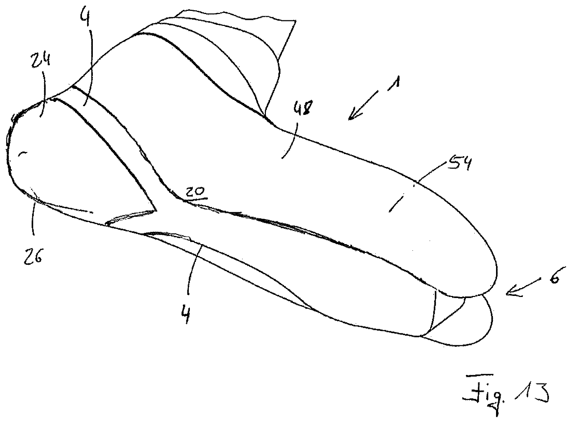

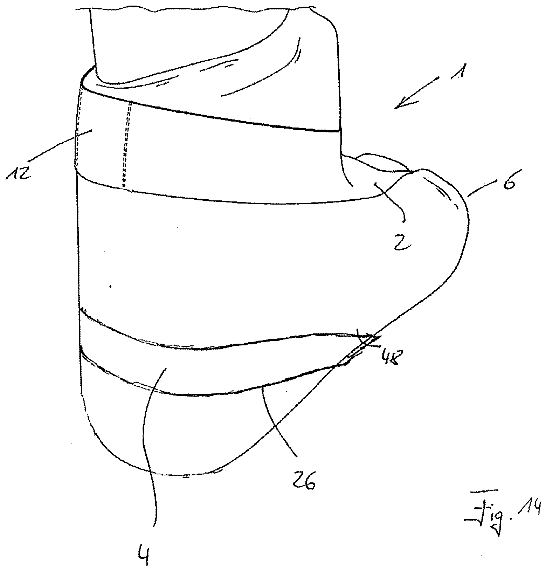

In den Figuren 12 bis 14 ist eine Ausgestaltung der Fußheberorthese 1 dargestellt, die der in den Figuren 9 bis 11 dargestellten Ausgestaltung mit dem Strumpf 48 entspricht. Auch hier ist das erste Zugelement 2 zu erkennen, das sich über den Fußrücken 10 erstreckt. Allerdings verfügt das in den Figuren 12 bis 14 gezeigte Ausführungsbeispiel auch über ein zweites Zugelement 4, das sich um die Ferse 24, die durch die gebildete Öffnung 26 hindurchragt, erstreckt.

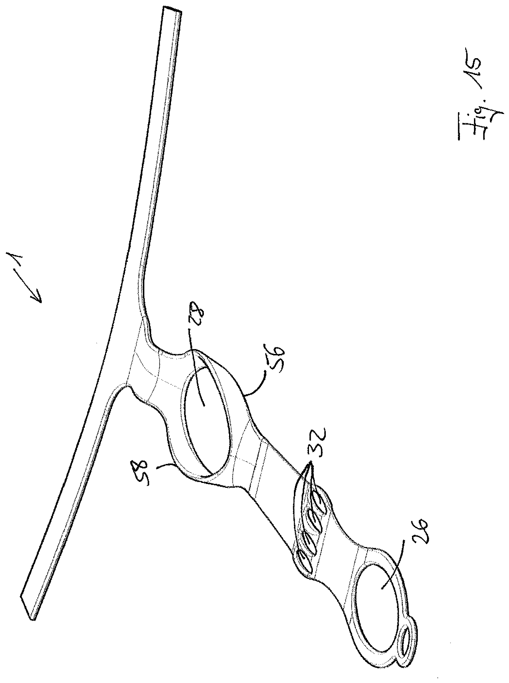

Figur 15 zeigt ein weiteres Ausführungsbeispiel der Fußheberorthese 1. Auch sie verfügt über die Öffnung 26, durch die die Ferse im angelegten Zustand herausragt. Man erkennt zudem vier Öffnungen 32 für die Zehen sowie die Öffnung 28, durch die der Fuß beim Anlegen hindurchgeführt wird. Hauptunterschied zu der in Figur 6 gezeigten Ausführungsform ist die Ausgestaltung der Fußheberorthese 1 im Bereich der Öffnung 28. Sie ist nicht vollständig flach ausgebildet, sondern verfügt über eine dreidimensionale Kontur mit einem gegenüber einem unteren Rand 56 erhaben angeordneten oberen Rand 58. Diese zwischenverlaufende Wandung ist an die Geometrie des Beins angepasst, das durch die Öffnung 28 hindurchführt, wenn die Fußheberorthese 1 angelegt ist.

### Bezugszeichenliste

- 1: Fußheberorthese
- 2: erstes Zugelement
- 4: zweites Zugelement
- 6: Vorfußbereich
- 8: Fuß
- 10: Fußrücken
- 12: erste Lagerposition
- 14: Haltesteg
- 16: Unterschenkel
- 18: Umfassung
- 20: Fußsohle

- 22: zweite Lagerposition
- 24: Ferse
- 26: Öffnung
- 28: Öffnung
- 30: Verschlusselement

- 32: Öffnung
- 34: Zeh
- 36: Ballen
- 38: Lasche
- 40: Verstärkungselement

- 42: Umschließung
- 44: Formschlusselement
- 46: Schlitz
- 48: Strumpf
- 50: Bahn

- 52: Stabilisierungselement
- 54: Verbreiterung
- 56: unterer Rand
- 58: oberer Rand

## Patentansprüche

1. Fußheberorthese (1) mit
wenigstens einem ersten Zugelement (2), das eingerichtet ist,
sich im angelegten Zustand der Fußheberorthese (1) von einem Vorfußbereich (6) eines Fußes (8) entlang eines Fußrückens (10) des Fußes (8) zu erstrecken und
eine erste Zugkraft auf den Vorfußbereich (6) in Richtung auf eine erste Lagerposition (12) oberhalb eines oberen Knöchelgelenkes auszuüben, **dadurch gekennzeichnet, dass** die Fußheberorthese wenigstens ein zweites Zugelement (4) aufweist, das eingerichtet ist,
im angelegten Zustand der Fußheberorthese (1) eine zweite Zugkraft auf den Vorfußbereich (6) in Richtung auf eine zweite Lagerposition (22) in einem Fersenbereich (24) des Fußes (8) auszuüben.

2. Fußheberorthese (1) nach Anspruch 1, **dadurch gekennzeichnet, dass** das zweite Zugelement (4) eingerichtet ist, sich im angelegten Zustand der Fußheberorthese (1) von dem Vorfußbereich (6) entlang einer Fußsohle (20) des Fußes (8) zu erstrecken.

3. Fußheberorthese (1) nach Anspruch 1 oder 2, **dadurch gekennzeichnet, dass** das erste Zugelement (2) und das zweite Zugelement (4) im Vorfußbereich (6) miteinander verbunden sind und vorzugsweise einstückig miteinander ausgebildet sind.

4. Fußheberorthese (1) nach Anspruch 1, 2 oder 3, **dadurch gekennzeichnet, dass** das zweite Zugelement (4) eine Öffnung (26) aufweist, so dass es um die Ferse (24) des Fußes (8) herumlegbar ist.

5. Fußheberorthese (1) nach einem der vorstehenden Ansprüche, **dadurch gekennzeichnet, dass** das erste Zugelement (2) wenigstens eine Öffnung (28) aufweist, so dass es um den Knöchelbereich herumlegbar ist.

6. Fußheberorthese (1) nach Anspruch 5, **dadurch gekennzeichnet, dass** die Fußheberorthese (1) wenigstens einen Haltesteg (14) aufweist, der sich im angelegten Zustand von der ersten Lagerposition (12) nach oben erstreckt und um den Unterschenkel (16) herumführbar ist.

7. Fußheberorthese (1) nach einem der vorstehenden Ansprüche, **dadurch gekennzeichnet, dass** sich zwischen dem ersten Zugelement (2) und dem zweiten Zugelement (4) wenigstens eine Öffnung (32) befindet, durch die sich im angelegten Zustand wenigstens ein Zeh (34) des Fußes (8) erstreckt.

8. Fußheberorthese (1) nach einem der vorstehenden Ansprüche, **dadurch gekennzeichnet, dass** die Fußheberorthese (1) wenigstens ein Verstärkungselement (40) aufweist, das an dem Unterschenkel (16) befestigbar ist und entlang des Fußrückens (10) verläuft, so dass die von dem ersten Zugelement (2) aufbringbare erste Zugkraft verstärkbar ist.

9. Fußheberorthese (1) nach einem der vorstehenden Ansprüche, **dadurch gekennzeichnet, dass** die Fußheberorthese (1) wenigstens ein Stabilisierungselement aufweist, das sich im angelegten Zustand um den Vorfußbereich (6) herum erstreckt.

10. Fußheberorthese (1) nach einem der vorstehenden Ansprüche, **dadurch gekennzeichnet, dass** die Fußheberorthese (1) aus Silikon besteht und vorzugsweise einstückig ausgebildet ist.

11. Fußheberorthese (1) nach einem der Ansprüche 1 bis 9, **dadurch gekennzeichnet, dass** die Fußheberorthese (1) einen Strumpf aus einem textilem Material aufweist, an dem das erste Zugelement (2) und das zweite Zugelement (4) angeordnet, bevorzugt angeklebt oder angespritzt, sind.

## Claims

1. Foot lift orthosis (1) comprising
at least a first tension element (2) designed to extend from a frontal region (6) of a foot (8) along an instep (10) of the foot (8) when the foot lift orthosis (1) is being worn, and to exert, on the frontal foot region (6), a first tensile force toward a first support position (12) above an upper ankle joint,
**characterized in that** the foot lift orthosis further comprises
at least a second tension element (4) designed to exert, on the frontal foot region (6), a second tensile force toward a second support position (22) in a heel region (24) of the foot (8) when the foot lift orthosis (1) is being worn.

2. Foot lift orthosis (1) according to claim 1, **characterized in that** the second tension element (4) is designed to extend from the frontal foot region (6) along a sole (20) of the foot (8) when the foot lift orthosis (1) is being worn.

3. Foot lift orthosis (1) according to either claim 1 or claim 2, **characterized in that** the first tension element (2) and the second tension element (4) are interconnected in the frontal foot region (6) and are preferably integral with one another.

4. Foot lift orthosis (1) according to any of claims 1, 2 or 3, **characterized in that** the second tension element (4) has an opening (26) such that said element can be wrapped around the heel (24) of the foot (8).

5. Foot lift orthosis (1) according to any of the preceding claims, **characterized in that** the first tension element (2) has at least one opening (28) such that said element can be wrapped around the ankle region.

6. Foot lift orthosis (1) according to claim 5, **characterized in that** the foot lift orthosis (1) comprises at least one supporting link (14) that extends upward from the first support position (12) and can be guided around the lower leg (16) when the orthosis is being worn.

7. Foot lift orthosis (1) according to any of the preceding claims, **characterized in that** at least one opening (32) through which at least one toe (34) of the foot (8) extends when the foot lift orthosis is being worn is located between the first tension element (2) and the second tension element (4).

8. Foot lift orthosis (1) according to any of the preceding claims, **characterized in that** the foot lift orthosis (1) comprises at least one reinforcement element (40) that can be fastened on the lower leg (16) and extends along the instep (10) such that the first tensile force that can be applied by the first tension element (2) can be increased.

9. Foot lift orthosis (1) according to any of the preceding claims, **characterized in that** the foot lift orthosis (1) comprises at least one stabilizing element that extends around the frontal foot region (6) when the foot lift orthosis is being worn.

10. Foot lift orthosis (1) according to any of the preceding claims, **characterized in that** the foot lift orthosis (1) is made of silicone and is preferably formed in one piece.

11. Foot lift orthosis (1) according to any of claims 1 to 9, **characterized in that** the foot lift orthosis (1) comprises a stocking made of a textile material on which the first tension element (2) and the second tension element (4) are arranged, preferably bonded or molded.

## Revendications

1. Orthèse lève-pied (1), comportant
au moins un premier élément de traction (2) qui est conçu pour dans l'état appliqué de l'orthèse lève-pied (1), s'étendre à partir d'une région d'avant-pied (6) d'un pied (8) le long du cou-de-pied (10), et pour exercer une première force de traction sur la zone d'avant-pied (6) en direction d'une première position d'appui (12) au-dessus d'une articulation supérieure de la cheville,
**caractérisée en ce que**
l'orthèse lève-pied comprend au moins un second élément de traction (4) qui est conçu pour, dans l'état appliqué de l'orthèse lève-pied (1), exercer une seconde force de traction sur la zone d'avant-pied (6) en direction d'une seconde position d'appui (22) dans une région de talon (24) du pied (8).

2. Orthèse lève-pied (1) selon la revendication 1,
**caractérisée en ce que**
le second élément de traction (4) est conçu pour, dans l'état appliqué de l'orthèse lève-pied (1), s'étendre depuis la zone d'avant-pied (6) le long d'une plante (20) du pied (8).

3. Orthèse lève-pied (1) selon la revendication 1 ou 2,
**caractérisée en ce que**
le premier élément de traction (2) et le second élément de traction (4) sont reliés l'un à l'autre et sont de préférence réalisés d'un seul tenant l'un avec l'autre, dans la zone d'avant-pied (6).

4. Orthèse lève-pied (1) selon la revendication 1, 2 ou 3,
**caractérisée en ce que**
le second élément de traction (4) présente une ouverture (26) de manière à pouvoir être posé autour du talon (24) du pied (8).

5. Orthèse lève-pied (1) selon l'une des revendications précédentes,
**caractérisée en ce que**
le premier élément de traction (2) présente au moins une ouverture (28) de manière à pouvoir être posé autour de la zone de cheville.

6. Orthèse lève-pied (1) selon la revendication 5
**caractérisée en ce que**
l'orthèse lève-pied (1) comprend au moins une barrette de retenue (14) qui, dans l'état appliqué, s'étend depuis la première position d'appui (12) vers le haut et peut être menée autour du bas de jambe (16).

7. Orthèse lève-pied (1) selon l'une des revendications précédentes,
**caractérisée en ce que**
au moins une ouverture (32) se situe entre le premier élément de traction (2) et le second élément de traction (4), ouverture à travers laquelle s'étend au moins un orteil (34) du pied (8), dans l'état appliqué.

8. Orthèse lève-pied (1) selon l'une des revendications précédentes,
**caractérisée en ce que**
l'orthèse lève-pied (1) comprend au moins un élément de renforcement (40) qui peut être fixé au bas de jambe (16) et qui s'étend le long du cou-de-pied (10), de sorte que la première force de traction susceptible d'être appliquée par le premier élément de traction (2) peut être amplifiée.

9. Orthèse lève-pied (1) selon l'une des revendications précédentes,
**caractérisée en ce que**
l'orthèse lève-pied (1) comprend au moins un élément de stabilisation qui, dans l'état appliqué, s'étend autour de la zone d'avant-pied (6).

10. Orthèse lève-pied (1) selon l'une des revendications précédentes,
**caractérisée en ce que**
l'orthèse lève-pied (1) est constituée en silicone et est réalisée de préférence d'un seul tenant.

11. Orthèse lève-pied (1) selon l'une des revendications 1 à 9,
**caractérisée en ce que**
l'orthèse lève-pied (1) comprend une chaussette en un matériau textile sur laquelle le premier élément de traction (2) et le second élément de traction (4) sont agencés, de préférence par collage ou par projection.
